# EUROPÄISCHE PATENTSCHRIFT

(11) **EP 0 987 527 B1**
(45) Veröffentlichungstag und Bekanntmachung des Hinweises auf die Patenterteilung: **07.06.2006**
(21) Anmeldenummer: 99112469.4
(22) Anmeldetag: 28.07.1998
(51) Int. Cl.: G01G 17/00, G01N 33/00, G01N 33/15

(54) **Tablettentestgerät**
Testing device for tablets
Appareil de contrôle des comprimés

(30) Priorität: 30.07.1997 DE 19733436
(43) Veröffentlichungstag der Anmeldung: 22.03.2000
(62) Teilanmeldung aus: 98114090.8
(73) Patentinhaber: ERWEKA GmbH, D-63150 Heusenstamm (DE)
(72) Erfinder: Müller, Werner G., 60323 Frankfurt/Main (DE)
(74) Vertreter: Kirschner, Klaus Dieter

(56) Entgegenhaltungen:
- WO-A-90/01010
- DE-U- 8 402 581
- US-A- 4 884 463
- PATENT ABSTRACTS OF JAPAN vol. 1995, no. 11, 26. Dezember 1995 (1995-12-26) & JP 07 206144 A (IKEGAMI TSUSHINKI CO LTD), 8. August 1995 (1995-08-08)
- Bedienungsanleitung zum Tablettentestgerät RQ 100 in der Fassung vom 20.07.1993

## Beschreibung

Die Erfindung betrifft ein Tablettentestgerät zum Testen von Oblong-Tabletten oder ähnlich geformten Tabletten, insbesondere zum Wiegen, Vermessen und Bruchtesten derartiger Tabletten, in entsprechenden Teststationen nach dem Oberbegriff des Anspruchs 1.

Aus dem Deutschen Gebrauchsmuster 8 402 581 ist ein Tablettenprüfgerät mit einer Transporteinrichtung bekannt, bei dem die Tabletten von einer Zufuhreinrichtung auf eine Transporteinrichtung mit einem Rechen gelangen, der mehrere Gabeln aufweist, die die Tabletten auf einer Transportbahn weiterbewegen. Eine Bewegungseinrichtung für den Rechen transportiert den Rechen um eine Strecke in Richtung der Transportrichtung, hebt dann den Rechen an und führt ihn in angehobenem Zustand entgegen der Transportrichtung um eine entsprechende Strecke wieder zurück. Anschließend wird der Rechen abgesenkt und erneut um eine Transportstrecke weiterbewegt. Entlang der Bewegungsbahn des Rechens sind mehrere Teststationen, nämlich eine Waage, eine Teststation für die Dickenmessung und eine Teststation für den Bruchtest vorgesehen. Das bekannte Tablettentestgerät ist für runde oder kugelförmige Tabletten geeignet, weniger jedoch für sogenannte Oblong-Tabletten und ähnlich geformte Tabletten, die eine im wesentlichen ovale Form in Draufsicht, einen flachen Steg an ihrem Umfang sowie eine gewölbte Ober- und Unterseite aufweisen können. Derartige Tabletten bereiten auf dem bekannten Gerät Schwierigkeiten. Sie können in verschiedenen Teststationen, wie der Dickenmeßstation, der Durchmessermeßstation und der Station zur Bruchhärteprüfung nicht immer in der erforderlichen Weise ausgerichtet werden.

Aus PATENT ABSTRACTS OF JAPAN Bd. 095, Nr. 011, vom 26. Dezember 1995 und JP 07 206144 A ist eine Transporteinrichtung für Oblong-Tabletten nach dem Oberbegriff des Anspruchs 1 bekannt.

Aus der Bedienungsanleitung des Tablettentestgerätes RQ 100 der Firma Kilian GmbH & Co. KG ist ein Gerät bekannt, mit dem Gewicht, Dicke und Härte von Tabletten gemessen werden kann. Die Tabletten werden von einem Fördertopf auf eine Waagschale gegeben und von dort über eine Tablettenenutsche auf einem Drehteller zur Dicken- und Härtemessung transportiert. Die Drehtellerschale weist zwei Schikanenelemente auf, die die Tabletten in die richtige Meßlage ausrichten, aber diese Schikanenelemente sind nicht verschiebbar.

Der Erfindung liegt demgegenüber die Aufgabe zugrunde, ein Tablettentestgerät zum Testen von Oblong-Tabletten oder ähnlich geformten Tabletten, in entsprechenden Teststationen bereitzustellen, mit dem mehrere Tests, beispielsweise das Wiegen, Vermessen und Bruchtesten in entsprechenden Teststationen durchgeführt werden konnen, wobei die einzelnen Oblong-Tabletten oder ähnlich geformte Tabletten in den Teststationen mit der erforderlichen Genauigkeit positioniert werden können.

Zur Lösung dieser Aufgabe ist das erfindungsgemäße Tablettentestgerät dadurch gekennzeichnet, daß die Schikane als über ein quer zur Transporteinrichtung vertikal verfahrbares Schikanenelement ausgebildet ist, das zur Bildung eines Stolpersteines über die Transportebene hinausgefahren werden kann und zur Bildung einer Stolpernut über die Transportebene zurückgezogen werden kann. Es sind auch Zwischenpositionen des Schikanenelements, beispielsweise bündig mit der Transportebene denkbar, wenn ein Kippen der Oblong-Tabletten nicht erforderlich ist. Die Höhe oder Tiefe des Schikanenelements kann auf diese Weise innerhalb der Grenzen der maximalen Verfahrbarkeit desselben individuell auch zur Prüflingsform gewählt werden.

Durch diese Ausgestaltung des erfindungsgemäßen Tablettentestgeräts kann sichergestellt werden, daß vergleichbare Ergebnisse beim Testen der Tabletten, in den einzelnen Teststationen erhalten werden. Die Dicke der Oblong-Tabletten oder ähnlicher Tabletten ist definiert als die Dicke der Tablette senkrecht zu ihrer Längsachse und in der Mitte desselben. Daher wird die Dicke gemessen, indem die Tablette auf eine seiner gewölbten Flächen gelegt und mit einem Taster gegen die gegenüberliegende gewölbte Fläche gefahren wird, um den Abstand zwischen dem Taster und der Auflagefläche zu messen. Daher muß die Tablette entsprechend positioniert sein, um diese Dickenmessung durchführen zu können. Die Länge einer Oblong-Tablette oder einer ähnlich geformten Tablette und die Bruchhärte werden entlang der Längsachse gemessen beziehungsweise überprüft, so daß auf die Längsachse in einer bestimmten Orientierung in der entsprechenden Teststation angeordnet sein muß. Diese Erfordernisse können bei der erfindungsgemäßen Einrichtung leicht erfüllt werden, weil die Oblong-Tabletten oder ähnlichen Tabletten die richtige Orientierung spätestens dann haben, wenn sie über die Schikane gelaufen sind.

Eine vorteilhafte Ausgestaltung des erfindungsgemäßen Tablettentestgeräts ist dadurch gekennzeichnet, daß die Zufuhreinrichtung zur Zufuhr einzelner Tabletten an eine Waage, deren Waagschale an einen in Transportrichtung geneigten Boden in Form einer Rinne aufweist und oberhalb der Transporteinrichtung angeordnet ist, auf die die Tabletten von der Waagschale mit ihrer Längsachse parallel zur Längsrichtung der Rinne abgelegt werden. Damit wird eine Vororientierung der Tabletten erreicht, bevor sie auf der Transporteinrichtung endgültig positioniert werden.

Eine vorteilhafte Ausgestaltung des erfindungsgemäßen Tablettentestgeräts ist dadurch gekennzeichnet, daß die Zufuhreinrichtung eine in Transportrichtung geneigte Zufuhrrinne aufweist, in die die Tabletten insbesondere von einem Vibrations-Vereinzler eingespeist werden. In der Zufuhrrinne kann bereits eine gewisse Vororientierung der Tabletten erfolgen.

Eine weitere vorteilhafte Ausgestaltung des erfindungsgemäßen Tablettentestgeräts ist dadurch gekennzeichnet, daß zwischen der Waage und der Transporteinrichtung eine flache oder profilierte Übergaberinne vorgesehen ist, so daß in vorteilhafter Weise die Orientierung der Tabletten erhalten bleibt, die sie in der Waage erhalten haben.

Eine weitere vorteilhafte Ausgestaltung des erfindungsgemäßen Tablettentestgerätes ist dadurch gekennzeichnet, daß der Boden der Waagschale einen V-förmigen Querschnitt hat. Mit dieser Ausgestaltung der Rinne ist sichergestellt, daß die Tabletten mit ihrer Längsachse im V-förmigen Querschnitt des Bodens der Waagschale ausgerichtet werden, wodurch eine optimale Orientierung der Oblong-Tablette erreicht wird.

Eine weitere vorteilhafte Ausgestaltung des erfindungsgemäßen Testgerätes ist dadurch gekennzeichnet, daß die Zufuhrrinne und/oder die Übergaberinne ebenfalls einen V-förmigen Querschnitt wie die Rinne der Waagschale aufweisen.

Schließlich ist eine weitere vorteilhafte Ausgestaltung des erfindungsgemäßen Tablettentestgerätes dadurch gekennzeichnet, daß die Transporteinrichtung eine Transportschiene und einen Rechen aufweist, der die Tabletten auf der Transportschiene vorwärts bewegt. Damit kann sichergestellt werden, daß die Orientierung der Oblong-Tablette, wenn sie von der Waage kommt, während des Weitertransports erhalten bleibt.

Ein Ausführungsbeispiel der Erfindung wird nun anhand der beiliegenden Zeichnung beschrieben. Es zeigen:
- Figur 1: eine schematische Seitenansicht des oberen Teils eines Tablettentest gerätes; und
- Figur 2: eine teilweise Draufsicht auf das Tablettentestgerät nach Figur 1; und
- Figur 3: eine Seitenansicht eines Details des Tablettentestgerätes nach Figur 1.

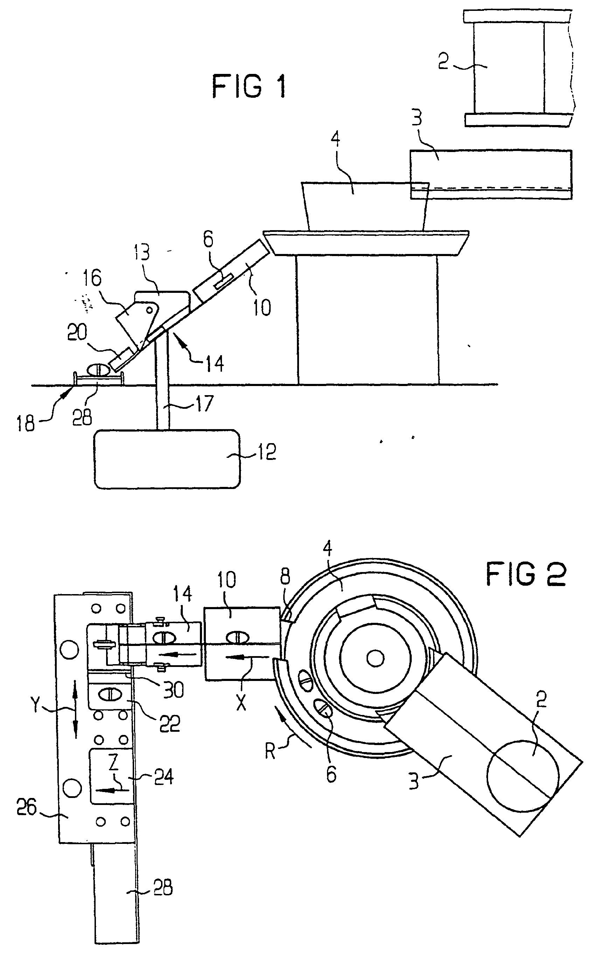

Wie aus den Figuren 1 und 2 zu ersehen ist, weist das Tablettentestgerät ein Magazin 2 beziehungsweise einen Vorratsbehälter für Tablettten auf. Von dem Magazin 2 gelangen die Oblong-Tabletten in ein Vibrations-Vereinzlungssystem, das aus einer Längsförderrinne 3 und einem Rundförderer 4 besteht. Sowohl die Längsförderrinne 3 als auch der Rundförderer 4 können in bestimmten Geräteversionen entfallen. Von dem Rundförderer 4 werden die Tabletten 6 in Richtung des Pfeiles R (Figur 2) transportiert und über eine Abgabeöffnung 8 in eine Zufuhrrinne 10 abgegeben, die zu der Zufuhreinrichtung gehört und in Transportrichtung (Pfeil X, Figur 2) von der Auslaßöffnung 8 des Rundförderers 4 nach unten geneigt ist. Von der Zufuhrrinne 10 werden die Tabletten an eine Waage 12 übergeben, deren Waagschale 13 einen in Transportrichtung geneigten Boden 14 in Form einer Rinne aufweist. Die Waagschale 13 wird an ihrem unteren Ende von einer Klappe 16 verschlossen, die aus dem Weg der Tablette heraus bewegt werden kann, so daß diese die Waagschale 13 verlassen kann. Die Waagschale 13 mit dem Boden 14 und der Klappe 16 ist über einen Verbindungsstößel 17 mit der Waage 12 verbunden.

Zwischen der Waage 12 und einer Transporteinrichtung für die Oblong-Tabletten ist eine Übergaberinne 20 vorgesehen.

Die Waagschale 13 hat an ihrem Boden 14 eine V-förmigen Querschnitt. Die Zufuhrrinne 10 und/oder die Übergaberinne 20 können einen entsprechenden V-förmigen Querschnitt oder auch eine ebene Form aufweisen.

Die Transporteinrichtung 18 ist unterhalb der Waagschale 13 und unterhalb der Übergaberinne 20 vorgesehen, und die Oblong-Tabletten werden von der Waagschale 13 mit ihrer Längsachse parallel zur Längsrichtung der Rinne in dem Boden 14 der Waagschale abgelegt. Die Transporteinrichtung, die aus einer Transportschiene 28 und einem Rechen 26 besteht, transportiert die Tabletten in dieser Ausrichtung durch weitere Teststationen, insbesondere eine Teststation 22 für die Dickenmessung, in der ein Dickenmessungs-Sensor von oben an die Tablette herangefahren wird, und eine Teststation 24 in der der Durchmesser und die Bruchhärte der Tabletten gemessen wird. Der Doppelpfeil Y deutet an, daß der Rechen 26 sich über der Transportschiene 28 (Figur 1) hin und her bewegt, um die Oblong-Tabletten durch die Teststation zu transportieren. In dem Teil der Teststation 24, in dem der Bruchhärtetest durchgeführt wird, wirkt ein Bruchbacken (nicht gezeigt) in Richtung des Pfeiles Z auf die Oblong-Tabletten in deren Längsrichtung.

Zwischen der Stelle, an der die Oblong-Tabletten von der Waagschale 13 kommend auf die Transporteinrichtung 18 übergeben werden, und den weiteren Teststationen 22, 24 ist ein Stolperstein beziehungsweise ein Keil 30 in der Transporteinrichtung angeordnet, der eventuell hochkant auf ihrem Steg stehende Oblong-Tabletten kippt, so daß sie auf ihre runde Seite fallen.

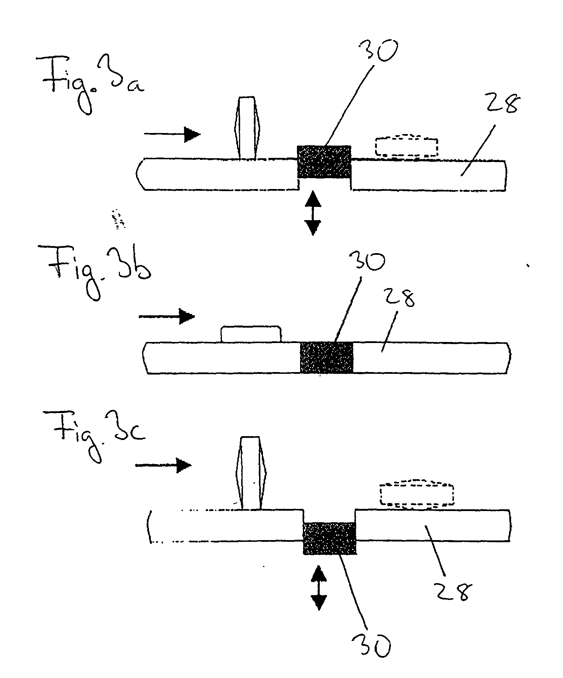

Figur 3 zeigt eine Ausführungsform des Tablettentestgerätes nach Figur 1, bei dem die Stolpereinrichtung als vertikal verfahrbares Schikanenelement 30 ausgebildet ist, das quer zur Transportrichtung ausgerichtet ist und das zur Bildung eines Stolpersteines über die Transportebene hinausgefahren werden kann und zur Bildung einer Stolpernut unter die Transportebene zurückgezogen werden kann. Figur 3a zeigt das Schikanenelement 30 in einer über die Transportebene hinausgefahrenen Position zur Bildung eines Stolpersteines, Figur 3b zeigt das Schikanenelement 30 in einer Position bündig mit der Transporteinrichtung 28 und Figur 3c zeigt das Schikanenelement 30 in einer unter die Transportebene zurückgezogenen Position zur Bildung einer Stolpernut

Aus der vorhergehenden Beschreibung ist ersichtlich, daß die Oblong-Tabletten oder ähnliche Tabletten in den Teststationen 22, 24, wo es auf die genaue Orientierung der Oblong-Tabletten ankommt, mit der richtigen Orientierung positioniert werden.

## Patentansprüche

1. Tablettentestgerät zum Testen von Oblong-Tabletten oder ähnlich geformten Tabletten, insbesondere zum Wiegen, Vermessen und Bruchtesten derartiger Tabletten, in entsprechenden Teststationen (22, 24) umfassend eine Transporteinrichtung (18), die die auf ihr abgelegten Tabletten durch die Teststationen (22, 24) transportiert, wobei zwischen der Stelle, an der die Oblong-Tabletten auf die Transporteinrichtung (18) übergeben werden, und den Teststationen (22, 24) eine Schikane vorgesehen ist, die eventuell hochkant auf ihrem Steg stehende Oblong-Tabletten kippt, so daß sie auf ihre runde Seite fallen, **dadurch gekennzeichnet, daß** die Schikane als über ein quer zur Transporteinrichtung vertikal verfahrbares Schikanenelement(30) ausgebildet ist, das zur Bildung eines Stolpersteines über die Transportebene hinausgefahren werden kann und zur Bildung einer Stolpernut über die Transportebene zurückgezogen werden kann.

2. Tablettentestgerät nach Anspruch 1, **gekennzeichnet durch** eine Zufuhreinrichtung (4, 10) zur Zufuhr einzelner Tabletten (6) an eine Waage (12), deren Waagschale (13) einen in Transportrichtung geneigten Boden (14) in Form einer Rinne aufweist und oberhalb der Transporteinrichtung (18) angeordnet ist, auf die die Tabletten von der Waageschale (13) mit ihrer Längsachse parallel zur Längsrichtung der Rinne abgelegt werden.

3. Tablettentestgerät nach Anspruch 2, **dadurch gekennzeichnet, daß** die Zufuhreinrichtung eine zur Transporteinrichtung (18) geneigte Zufuhrrinne (10) aufweist, in die die Tabletten, insbesondere von einem Vibrations-Vereinzler, eingespeist werden.

4. Tablettentestgerät nach Anspruch 2 oder 3, **dadurch gekennzeichnet, daß** zwischen der Waage (12) und der Transporteinrichtung (18) eine Übergaberinne (20) vorgesehen ist.

5. Tablettentestgerät nach einem der Ansprüche 2 bis 4, **dadurch gekennzeichnet, daß** der Boden der Waagschale (13) einen V-förmigen Querschnitt hat.

6. Tablettentestgerät nach einem der Ansprüche 2 bis 5, **dadurch gekennzeichnet, daß** die Zufuhrrinne (10) und/oder die Übergaberinne (20) ebenfalls einen V-förmigen Querschnitt wie die Rinne der Waage (12) aufweist.

7. Tablettentestgerät nach einem der Ansprüche 2 bis 6, **dadurch gekennzeichnet, daß** die Transporteinrichtung (18) eine Transportschiene (28) und einen Rechen (26) aufweist, der die Tabletten auf der Transportschiene (28) vorwärts bewegt. ,

## Claims

1. A device for testing oblong tablets or similarly formed tablets, especially for weighing, measuring and fracture testing of such tablets, in suitable testing stations (22.24) comprising conveying means (18) which transport the tablet deposited thereon through test the testing stations (22,24), where in between the position where the oblong tablets are handed over to the transport means (18) and the testing stations (22,24) a toppling means (30) is provided, which topples over any oblong tablets standing on their edges so that same fall onto their round side, **characterized in that** the toppling means is built in the form of a toppling element (30) movable vertically transversely to the transport direction, which element may be moved beyond the transport plane to form a toppling stone and may be retracted below the transport plane to from the toppling recess.

2. The device of claim 1, **characterized by** feeding means (4,10) for feeding individual tablets (6) to a weighing means (12), whose weighing pan (13) possesses a floor (14) inclined in the conveying direction in the form of a trough and arranged above the conveying means (18), on which pan the tablets are deposited from the weighing pan (13) with their longitudinal axis parallel to the longitudinal direction of the trough.

3. The device of claim 2, **characterized in that** the feed means possesses a feed trough (10) inclined in relation to the conveying means (18), into which feed trough the tablets are fed, more particularly by a vibratory individualising means.

4. The device of claim 2 or 3, **characterized in that** in between the weighing means (12) and the conveying means (18) a transfer trough (20) is arranged.

5. The device of any of the claims 2 to 4, **characterized in that** the floor of the weighing pan (13) possesses a V-shaped cross section.

6. The device of any of the claims 2 to 5, **characterized in that** the feed trough (10) and/or the transfer trough (20) also possess(es) a V-shaped cross section as the trough of the weighing means (12).

7. The device of any of the claims 2 to 6, **characterized in that** the conveying means (18) possesses a conveying rail (28) and a rake (26), which moves the tablets on the conveying rail (28) forward.

## Revendications

1. Appareil de contrôle des comprimés pour le contrôle des comprimés allongés ou autres comprimés de forme pareille, spécialement pour pesée, mesurage et contrôle à rupture de tels comprimés, dans les stations de contrôle (22, 24) comprenant un dispositif de transport (18), qui transporte par les stations de contrôle (22, 24) les comprimés assis sur celui, où, entre le lieu dans lequel les comprimés allongés sont transmis sur le dispositif de transport (18) et les stations de contrôle (22, 24), est pourvue une chicane, qui renverse les éventuels comprimés allongés étant assis sur le chant, de sorte que ceux-ci tombent sur leur côté rond, **caractérisé en ce que**, la chicane est formée comme un élément de chicanage (30) déplaçable verticalement, transversal à l'égard du dispositif de transport, qui, pour former un obstacle d'empêchement peut être retiré en dehors sur le plan de transport et pour former de cannelures d'empêchement peut être retiré sous le plan de transport.

2. Appareil de contrôle des comprimés selon la revendication 1, **caractérisé par** un dispositif d'alimentation (4, 10) pour l'alimentation de comprimés (6) singuliers, à une balance (12), dont le plateau de pesée (13) présente une base (14) inclinée dans la direction de transport, de la forme d'une auge, et est disposé au-dessus du dispositif de transport (18), sur lequel les comprimés sont déposés à partir du plateau de pesée (13) avec leur axe longitudinal parallèle à la direction longitudinale de l'auge.

3. Appareil de contrôle des comprimés selon la revendication 2, **caractérisé en ce que**, le dispositif d'alimentation présente une auge d'alimentation (10) pour le dispositif de transport (18), où sont introduits les comprimés, particulièrement par un séparateur par vibration.

4. Appareil de contrôle des comprimés selon la revendication 2 ou 3, **caractérisé en ce qu'**entre la balance (12) et le dispositif de transport (18) est pourvue une auge de transmission (20).

5. Appareil de contrôle des comprimés selon l'une des revendications 2 à 4, **caractérisé en ce que**, la base du plateau de pesée (13) présente une section transversale en forme de V.

6. Appareil de contrôle des comprimés selon l'une des revendications 2 à 5, **caractérisé en ce que**, l'auge d'alimentation (10) et/ou l'auge de transmission (20) présentent aussi une section transversale en forme de V, également comme l'auge de la balance (12).

7. Appareil de contrôle des comprimés selon l'une des revendications 2 à 6, **caractérisé en ce que**, le dispositif de transport (18) présente un rail de transport (28) et une grille (26), qui avance les comprimés sur le rail de transport (28).
